Europäisches Patentamt

European Patent Office

Office européen des brevets

(19)

(11) Veröffentlichungsnummer: **0 046 773**
**B1**

(12) ## EUROPÄISCHE PATENTSCHRIFT

(45) Veröffentlichungstag der Patentschrift:
05.12.84

(51) Int. Cl.³: **A 61 B 17/18**

(21) Anmeldenummer: 81900453.2

(22) Anmeldetag: **17.02.81**

(86) Internationale Anmeldenummer:
**PCT/EP 81/00014**

(87) Internationale Veröffentlichungsnummer:
**WO 81/02388 (03.09.81** Gazette 81/21)

(54) **VORRICHTUNG ZUM FIXIEREN EINES OBERSCHENKELHALSES AM SCHAFT EINES OBERSCHENKELKNOCHENS.**

(30) Priorität: **19.02.80 DE 3006518**

(43) Veröffentlichungstag der Anmeldung:
**10.03.82 Patentblatt 82/10**

(45) Bekanntmachung des Hinweises auf die Patenterteilung:
**05.12.84 Patentblatt 84/49**

(84) Benannte Vertragsstaaten:
**CH FR GB LI**

(56) Entgegenhaltungen:
**BE - A - 692 263**
**FR - A - 2 149 943**
**US - A - 3 604 414**

(73) Patentinhaber: **MECRON MEDIZINISCHE PRODUKTE GMBH, Nunsdorfer Ring 25-27, D-1000 Berlin 48 (DE)**

(72) Erfinder: **HANSLIK, Lothar, Leo-Baeck-Strasse 23, D-1000 Berlin 37 (DE)**

(74) Vertreter: **Jander, Dieter, Dipl.-Ing. et al, Dipl.-Ing. Dieter Jander Dr.-Ing. Manfred Böning Patentanwälte Kurfürstendamm 66, D-1000 Berlin 15 (DE)**

## Beschreibung

Die Erfindung bezieht sich auf eine Vorrichtung zum Fixieren des Oberschenkelhalses und des grossen Rollhügels am Schaft eines Oberschenkelknochens eines Menschen.

Die Fixierung kann notwendig werden zur Heilung eines Bruches, z.B. eines solchen Doppelbruches, bei dem sich der Rollhügel vom Hals und der Hals vom Schaft gelöst haben, aber auch im folgenden Fall: Es kommt vor, dass der Gelenkkopf in seinem Belastungsbereich (also beim stehenden Patienten oben) zerstört ist. Dann wird der Hals vom Schaft und vom Rollhügel getrennt und um 90° gedreht, derart, dass ein gesunder Bereich des Gelenkkopfes Belastungsbereich wird.

Bisher hat man im letzteren Falle den Schenkelhals und den grossen Rollhügel mittels Schrauben nach der Drehung an dem Knochenschaft fixiert.

Der Erfindung liegt die Aufgabe zugrunde, eine einfach zu handhabende und lange und sicher wirksame Fixierungsvorrichtung zu schaffen. Für den Fall der Anwendung der erfindungsgemässen Vorrichtung bei Drehung des Halses soll ausserdem eine einfachere Schnittführung durch den Knochen erreicht werden.

Diese Aufgabe wird erfindungsgemäss gelöst durch eine Winkelplatte, deren als Klingenteil ausgebildeter Schenkel axial in den Hals eingeschlagen wird und deren als Schaftabschnitt ausgebildeter andere Schenkel am Schaft befestigt wird und die den Hals am Schaft fixiert, und durch eine Krallenplatte, die am Schaftabschnitt der Winkelplatte und/oder am Schaft befestigt wird und den Rollhügel am Hals fixiert.

Eine in dieser Weise ausgebildete Vorrichtung lässt sich einfach handhaben, gewährleistet eine sicherere und haltbarere Fixierung der Knochenteile zueinander ohne die Gefahr des Lockerns nach gewisser Zeit als im bekannten Fall und gestattet bei Drehung des Halses zwecks Auswechslung des Gelenkkopfbelastungsbereiches eine einfachere Schnittführung durch den Knochen als im bekannten Fall.

Weitere Einzelheiten der Erfindung ergeben sich aus der Zeichnung.

Darin zeigen:

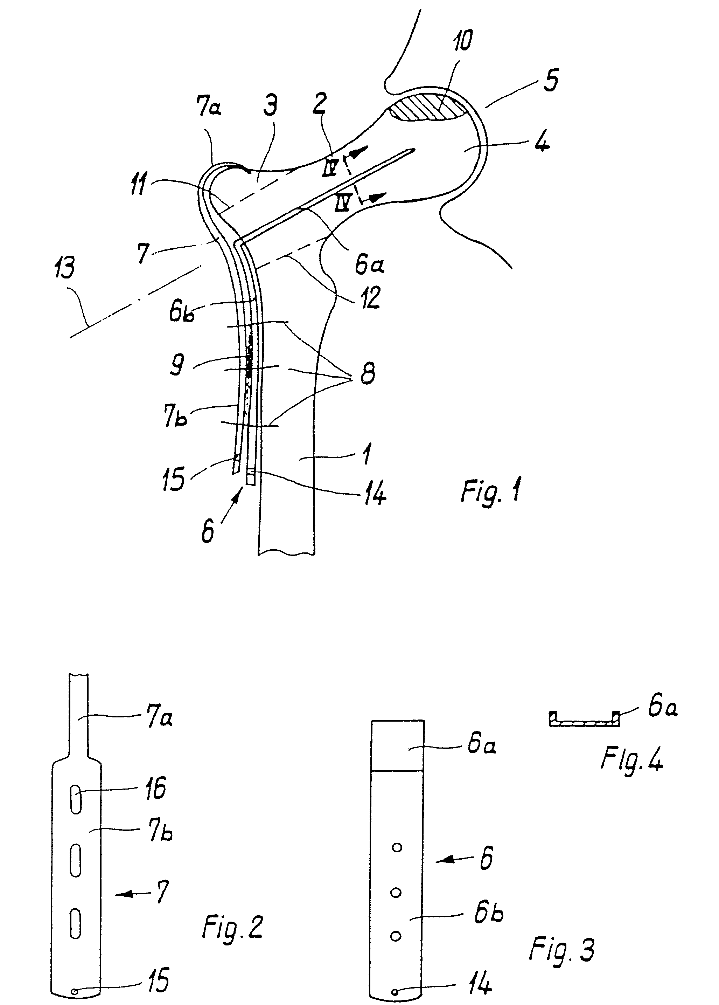

Fig. 1 eine Ansicht der erfindungsgemässen Vorrichtung und von Knochenteilen.
Fig. 2 eine Seitenansicht der Krallenplatte.
Fig. 3 eine Seitenansicht der Winkelplatte.
Fig. 4 einen Schnitt nach der Linie IV–IV der Fig. 1.
Fig. 5 eine der Fig. 1 entsprechende Ansicht bei Anwendung der Vorrichtung zwecks Heilung eines Bruches.

In den Figuren ist (sind) mit 1 ein Schaft eines Oberschenkelknochens, mit 2 dessen Hals, mit 3 ein grosser Rollhügel, mit 4 ein Oberschenkelkopf, mit 5 eine Hüftgelenkpfanne, mit 6 eine Winkelplatte, bestehend aus einem Klingenteil 6a und einem Schaftabschnitt 6b, mit 7 eine Krallenplatte, bestehend aus einem Schaftteil 7b und einem Haken 7a, mit 8 Knochenschrauben und mit 9 gegenläufige Zähne, die sich an der Krallenplatte 7 und dem Schaftabschnitt 6b befinden und eine Bewegung der Krallenplatte 7 nur in distaler Richtung gestatten, bezeichnet.

Zwecks Drehung des Halses 2 um 90°, um die zerstörte Stelle 10 aus dem Belastungsbereich zu entfernen und eine unzerstörte Stelle des Gelenkkopfes 4 in den Belastungsbereich zu bringen, wird der Klingenteil 6a in den Hals 2 eingeschlagen. Dann werden der grosse Rollhügel 3 vom Hals 2 und der Hals 2 vom Schaft 1 mittels zweier Schnitte 11 und 12 getrennt. Die Flächen, die nach der Drehung des Halses 2 aufeinander zu liegen kommen, werden präpariert, und der Hals 2 wird sodann unter Zuhilfenahme der Winkelplatte 6 gedreht. Nunmehr wird der Schaftabschnitt 6b der Winkelplatte 6 mitsamt dem Hals 2 mittels der Schrauben 8 an dem Schaft 1 fixiert. Damit die Flächen zwischen Hals 2 und Schaft 1 fest aufeinanderliegen, wird vorher zwischen dem Loch 14 des Schaftabschnittes 6b und dem Schaft 1 eine nicht dargestellte Spannvorrichtung angesetzt und mit dieser die entsprechende Flächenanpresskraft erzeugt. Sodann wird die Krallenplatte 7 angesetzt und mittels einer weiteren, nicht dargestellten Spannvorrichtung, die an einem Loch 15 und ebenfalls dem Schaft 1 angreift, gegen den Rollhügel 3 gezogen, derart, dass dieser gegen den Hals 2 gepresst wird. Die Zähne 9 bewirken, dass die Krallenplatte 7 zwar in Spannrichtung, aber nicht in entgegengesetzter Richtung bewegt werden kann. Anschliessend wird die Krallenplatte 7 mittels weiterer Schrauben 8 an dem Schaftabschnitt 6b bzw. dem Schaft 1 fixiert. Damit die Krallenplatte 7 angespannt werden kann, besitzt sie Langlöcher 16. Die allein im Schaftabschnitt 6b sitzenden Schrauben 8 sind in dem Schaftabschnitt 6b versenkt.

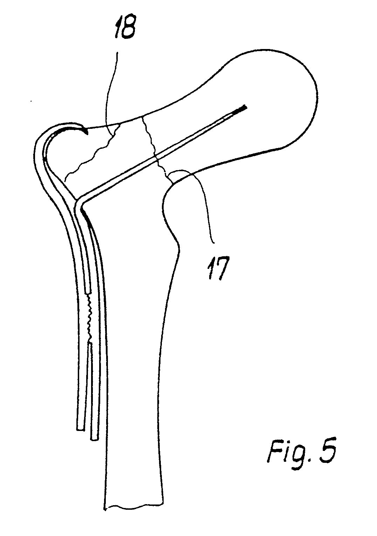

Fig. 5 zeigt die Anwendung der erfindungsgemässen Vorrichtung zwecks Heilung eines Bruches. Die Bruchflächen sind mit 17 und 18 bezeichnet.

## Patentansprüche

1. Vorrichtung zum Fixieren des Oberschenkelhalses und des grossen Rollhügels am Schaft eines Oberschenkelknochens, gekennzeichnet durch eine Winkelplatte (6), deren als Klingenteil (6a) ausgebildeter Schenkel axial in den Hals (2) eingeschlagen wird und deren als Schaftabschnitt (6b) ausgebildeter andere Schenkel am Schaft (1) befestigt wird und die den Hals (2) am Schaft (1) fixiert, und durch eine Krallenplatte (7), die am Schaftabschnitt (6b) der Winkelplatte (6) und/oder am Schaft (1) befestigt wird und den Rollhügel (3) am Hals (2) fixiert.

2. Vorrichtung nach Patentanspruch 1, dadurch gekennzeichnet, dass die Krallenplatte (7) auf dem Schaftabschnitt (6b) der Winkelplatte in Schaftlängsrichtung verschieblich ist und dass der Schaftabschnitt (6b) der Winkelplatte und die Krallenplatte (7) mittels Schrauben (8) aufeinanderliegend am Schaft (1) befestigbar sind.

3. Vorrichtung nach Patentanspruch 1 oder 2, gekennzeichnet durch Spannvorrichtungen, die an den distalen Enden des Schaftabschnittes (6b) der Winkelplatte bzw. der Krallenplatte (7) einerseits und dem Schaft (1) andererseits angreifen und mittels derer der Hals (2) gegen den Schaft (1) und der Rollhügel (3) gegen den Hals (2) gezogen werden können.

4. Vorrichtung nach einem oder mehreren der Patentansprüche 1 bis 3, gekennzeichnet durch Zähne (9), insbesondere gegenläufige Zähne, auf den aneinander anliegenden Flächen des Schaftabschnittes (6b) der Winkelplatte und der Krallenplatte (7), die eine Bewegung der Krallenplatte (7) gegenüber dem Schaftabschnitt (6b) der Winkelplatte nur in Spannrichtung ermöglichen.

5. Vorrichtung nach Anspruch 2, dadurch gekennzeichnet, dass die Krallenplatte (7) ein einziges Langloch oder mehrere Langlöcher (16) aufweist.

6. Vorrichtung nach einem oder mehreren der Ansprüche 1 bis 5, dadurch gekennzeichnet, dass die Krallenplatte (7) aus einem Schaftteil (7b) und einem den Rollhügel (3) berührend umlaufenden Haken (7a) besteht.

7. Vorrichtung nach Anspruch 6, dadurch gekennzeichnet, dass das freie Ende des Hakens (7a) spitz ist und beim Spannen in den Rollhügel (3) eindringt.

8. Vorrichtung nach Anspruch 6 oder 7, dadurch gekennzeichnet, dass der Haken (7a) in den Erstreckungen, die senkrecht zur Krümmungsebene (Fig. 1) liegen, schmäler als der Schaftteil (7b) der Krallenplatte (7) ist oder die gleiche Breite hat.

9. Vorrichtung nach einem oder mehreren der Ansprüche 1 bis 8, dadurch gekennzeichnet, dass die Köpfe der Schrauben (8), die nur den Schaftabschnitt (6b) durchdringen, in diesem versenkt sitzen.

## Claims

1. Device for fixing the femoral collum and the greater trochanter on the body of a femoral bone, characterized by an angular plate (6), the one leg of which, developed as sharpened portion (6a), is driven axially into the collum (2), and the other leg of which, developed as rod portion (6b), is secured on the body (1) fixing the collum (2) at the body (1), and by a claw-shaped plate (7), which is secured on the rod portion (6b) of the angular plate (6) and/or on the body (1) and fixing the trochanter (3) on the collum (2).

2. Device according to claim 1, characterized therein that the claw-shaped plate (7) is displaceable on the rod portion (6b) of the angular plate in longitudinal direction of the rod and that the rod portion (6b) of the angular plate and the claw-shaped plate (7) can be secured on the body (1) by means of screws (8) joining said plate and said body against each other.

3. Device as defined in claim 1 or 2, characterized by clamping devices engaging the distal ends of the rod portion (6b) of the angular plate or of the claw-shaped plate (7) on the one side and the body (1) on the other side, said clamping devices permitting tightening of the collum (2) against the body (1) and of the trochanter (3) against the collum (2).

4. Device as defined by any one or several of claims 1 to 3, characterized by teeth (9), in particular oppositely directed teeth on the adjacently arranged surfaces of the rod portion (6b) of the angular plate and the claw-shaped plate (7), said teeth permitting a motion of the claw-shaped plate (7) relative to the rod portion (6b) of the angular plate only in the direction of clamping.

5. Device as defined by claim 2, characterized by the fact that the claw-shaped plate (7) has one single elongated hole or a plurality of elongated holes (16).

6. Device as defined by any one or several of claims 1 to 5, characterized by the fact that the claw-shaped plate (7) consists of a rod portion (7b) and a hook (7a), said hook being in contact with and running around the trochanter (3).

7. Device as defined by claim 6, characterized by the fact that the free end of the hook (7a) is pointed and penetrates the trochanter (3) upon clamping.

8. Device as defined in claim 6 or 7, characterized by the fact that within the reaches extending perpendicular to the plane of curvature (Fig. 1), the width of the hook (7a) is less than or equal the width of the rod portion (7b) of the claw-shaped plate (7).

9. Device as defined by any one or several of claims 1 to 8, characterized by the fact that the heads of the screws (8) penetrating only the rod portion (6b) are disposed sunk in said portion.

## Revendications

1. Dispositif pour la fixation du col du fémur et du grand trochanter au corps d'un fémur, caractérisé par une plaque coudée (6) dont la branche formant lame (6a) est enfoncée axialement dans le col (2) et dont l'autre branche formant un segment de corps (6b) est fixée au corps (1), et qui fixe le col (2) au corps (1), et par une plaque à griffe (7) qui est fixée au segment de corps (6b) de la plaque coudée (6) et/ou au corps (1) et qui fixe le trochanter (3) au col (2).

2. Dispositif selon la revendication 1, caractérisé en ce que la plaque à griffe (7) peut se déplacer sur le segment de corps (6b) de la plaque coudée dans la direction longitudinale du corps et en ce que le segment de corps (6b) de la plaque coudée et la plaque à griffe (7) peuvent être fixés au corps (1) l'un appliqué sur l'autre au moyen de vis (8).

3. Dispositif selon la revendication 1 ou 2, caractérisé par des dispositifs de serrage qui attaquent les extrémités distales du segment de corps (6b) de la plaque coudée et de la plaque à griffe (7), d'une part, et le corps (1) d'autre part et au moyen desquels le col (2) peut être attiré vers le corps (1) et le trochanter (3) vers le col (2).

4. Dispositif selon une ou plusieurs des revendications 1 à 3, caractérisé par des dents (9), notamment des dents inclinées en sens inverse, prévues sur les surfaces du segment de corps (6b) de la plaque coudée et de la plaque à griffe (7) qui sont en contact entre elles, qui ne permettent un déplacement de la plaque à griffe (7) par rapport au segment de corps (6b) de la plaque coudée que dans le sens du serrage.

5. Dispositif selon la revendication 2, caractérisé en ce que la plaque à griffe (7) présente un trou allongé unique ou plusieurs trous allongés (16).

6. Dispositif selon une ou plusieurs des revendications 1 à 5, caractérisé en ce que la plaque à griffe (7) est composée d'une partie de corps (7b) et d'un crochet (7a) qui est recourbé et en contact avec le trochanter (3).

7. Dispositif selon la revendication 6, caractérisé en ce que l'extrémité libre du crochet (7a) est pointue et pénètre dans le trochanter (3) lors du serrage.

8. Dispositif selon la revendication 6 ou 7, caractérisé en ce que, dans les régions qui s'étendent perpendiculairement au plan de courbure (Fig. 1), le crochet (7a) est plus étroit que la partie de corps (7b) de la plaque à griffe (7) ou présente la même largeur.

9. Dispositif selon une ou plusieurs des revendications 1 à 8, caractérisé en ce que les têtes des vis (8) qui traversent uniquement le segment de corps (6b) sont disposées noyées dans ce segment.

0 046 773

Fig. 1

Fig. 2

Fig. 3

Fig. 4

5

Fig. 5